# EUROPEAN PATENT APPLICATION

(11) **EP 4 800 003 A1**
(43) Date of publication of application: **02.09.2026**
(21) Application number: 25160632.3
(22) Date of filing: 27.02.2025
(51) Int. Cl.: C07C 273/04, C07C 273/16, B01J 19/00

(54) **A METHOD FOR OPERATING A UREA PRODUCING PLANT**

(71) Applicant: Yara International ASA, 0277 Oslo (NO)
(72) Inventor: PORRO, Lino Giovanni, 0277 Oslo (NO)
(74) Representative: De Clercq & Partners

(57) **Abstract**

The present disclosure provides a method for operating a urea plant comprising an urea synthesis section, wherein the synthesis section comprises at least one high-pressure process line, a flushing system fluidly connected to the at least one high-pressure process line, a first high pressure flushing pump configured to direct an aqueous solution to the flushing system, and at least one sensor for measuring a process variable, wherein the urea plant comprises a second high-pressure flushing pump configured to direct an aqueous solution to the at least one sensor for measuring a process variable, and wherein the method further comprises continuously directing an aqueous solution from the second high-pressure flushing pump to the flushing system. The present disclosure further provides an assembly comprising a flushing system, a first high pressure flushing pump fluidly connected to the flushing system, a second high-pressure flushing pump and at least one sensor for measuring a process variable in the synthesis section, wherein the second high-pressure flushing pump is fluidly connected to the flushing system and to the at least one sensor and configured to provide an aqueous solution to the flushing system and to the at least one sensor.

## Description

### Field of the disclosure

The present disclosure belongs to the field of urea production.

### Background information

A urea producing plant comprises several sections: a urea synthesis section configured to receive ammonia and carbon dioxide and produce a synthesis solution comprising water, urea, ammonium carbamate, and free ammonia; a recovery section configured to process the synthesis solution into an aqueous urea solution, a concentration section configured to process the aqueous urea solution into a urea melt, a finishing section configured to process a urea melt into a final urea product, which may be liquid or solid.

The urea synthesis section comprises at least one device, the synthesis reactor, wherein carbon dioxide and ammonia are reacted under high temperature and pressure, typically exceeding 10 MPa, to produce an aqueous solution comprising urea, ammonium carbamate, and free ammonia.

Many urea plants further comprise a high-pressure stripper and a high-pressure carbamate condenser in their synthesis section. A stripper is device configured to receive an aqueous solution comprising urea, ammonium carbamate, and free ammonia and remove some of the ammonium carbamate and free ammonia from the aqueous solution. A carbamate condenser is a device configured to condense a gas stream comprising water, ammonia, and carbon dioxide, into an aqueous solution comprising ammonium salts. A synthesis section may also comprise pumps to direct the fluid compositions from one apparatus to another.

The synthesis section comprises high-pressure process lines that connect the various devices of the synthesis section with each other. Some of these high-pressure process lines comprise several valves connected to a flushing system, also called a flushing/draining system. When a urea plant needs to be stopped, either due to a planned maintenance or an unexpected shutdown, the synthesis section needs to be emptied: the solution comprised therein comprises ammonium carbamate, is highly corrosive and urea and/or ammonium carbamate may crystallize which may block the pipe and/or devices. For that purpose, a urea plant comprises a flushing system, comprising several pipes and valves, fluidly connected to the high-pressure process lines. The pipes of the flushing system are fluidly connected to a common header that is collecting the solution to be drained to a storage system or another section of the urea plant, in order to recover the reagents, i.e., ammonia and carbon dioxide, at the next start-up of the urea plant.
When the urea plant is in operation, i.e., producing urea, the flushing system should be kept isolated from the high-pressure process lines with the isolation valves being tightly closed.

Ammonium carbamate is a highly corrosive material, so equipment that is configured to contain aqueous solutions comprising ammonium carbamate at high temperatures and pressures have to use materials specifically designed for that purpose. Today, pipes with a good resistance to carbamate corrosion can be produced, but valves that connect these pipes are more challenging due to the more complicated design. Valves, in particular valves in the synthesis section, are considered today a weak point in a urea plant, and efforts to increase their reliability and lifespan are required. The flushing system, in particular the valves comprised therein, need to be regularly washed with water to ensure that no ammonium carbamate builds up due to small leakages of the valves. Ammonium carbamate build-up can crystallize, blocking the functionality of the system when needed and/or create corrosion in the flushing/draining pipes if stagnant. To avoid this risk, it is possible to keep the flushing system pressurized with water, at a pressure slightly higher than the synthesis pressure. By doing that, the water will enter the high-pressure process lines instead of the synthesis solution comprising ammonium carbamate going into the flushing system if one of more isolation valves in the synthesis section are leaking.

A urea plant also comprises at least one high-pressure water pump, configured to direct aqueous solutions, such as steam condensate, in different parts and equipment of the urea plant. One example is the high-pressure flushing pump, hereafter also referred to as the first high-pressure flushing pump, which is configured to inject water into the synthesis section once the section has been drained to remove any trace of urea and ammonium carbamate and to wash occasionally the draining/flushing system to check if any leakage from the valves is occurring. The first high-pressure flushing pump is configured to provide a very high flow, for example from 10 to 50 m³/h, at a high pressure, from 10.0 to 25.0 MPa, to be able to flush large pipes and devices, such as the synthesis reactor, the high-pressure stripper, and/or the high-pressure carbamate condenser. Conventionally, the first high-pressure flushing pump is configured to inject, continuously or occasionally, water into the flushing system of the synthesis section. A continuous injection ensures that a flushing system is kept free of ammonium carbamate solids, however, this requires a lot of energy by running the first high-pressure flushing pump in recycle mode, meaning that the water pumped by the pump is sent back to it, to supply from 10 to 50 thousand liters of water per hour into the synthesis section. A sporadic injection induces wear on the pump which has to be replaced more often, and therefore represents an added cost to the plant operations.

There is a need to develop a method which uses less water and power to maintain a flushing system in good condition, i.e., free of ammonium carbamate.

### Summary of the disclosure

Urea plants often comprise a second high-pressure pump, also referred to as a high-pressure instrumentation flushing pump. It has been found that this second high-pressure flushing pump can be used to provide water or process condensate to a draining system comprised in the synthesis section. This ensures that the draining system stays free of ammonium carbamate, reduces the power consumption of the urea plant and the wear of the first high-pressure flushing pump, which is only used during draining and flushing of the synthesis section.

In a first aspect, the present disclosure provides a method for operating a urea plant comprising a urea synthesis section, wherein the urea synthesis section comprises at least one high-pressure process line, a flushing system fluidly connected to the at least one high-pressure process line, a first high pressure flushing pump configured to direct an aqueous solution to the flushing system, at least one sensor for measuring a process variable, a second high-pressure flushing pump configured to direct an aqueous solution to the at least one sensor for measuring a process variable, characterized in that the method comprises continuously directing an aqueous solution from the second high-pressure flushing pump to the flushing system.

In another aspect, the present disclosure provides an assembly comprising a flushing system, a first high pressure flushing pump fluidly connected to the flushing system and configured to direct an aqueous solution to the flushing system, a second high-pressure flushing pump and at least one sensor for measuring a process variable in the synthesis section, wherein the flushing system comprises at least one pipe and at least one flow-regulating valve, and the second high-pressure flushing pump is fluidly connected to the flushing system and to the at least one sensor and configured to provide an aqueous solution to the flushing system and to the at least one sensor. In another aspect the present disclosure provides a urea plant comprising an assembly comprising a flushing system, a first high pressure flushing pump fluidly connected to the flushing system and configured to direct an aqueous solution to the flushing system, a second high-pressure flushing pump and at least one sensor for measuring a process variable in the synthesis section, wherein the flushing system comprises at least one pipe and at least one flow-regulating valve, and the second high-pressure flushing pump is fluidly connected to the flushing system and to the at least one sensor and configured to provide an aqueous solution to the flushing system and to the at least one sensor.

### Brief description of the figures

The following description of the figure of a specific embodiment of a system according to the present disclosure is only given by way of example and is not intended to limit the present explanation, its application or use. In the figure, identical reference numerals refer to the same or similar parts and features.
Figure 1 shows an assembly in a conventional urea plant comprising a flushing system according to the prior art.
Figure 2 shows an assembly in a urea plant comprising a flushing system for performing the method according to the present disclosure.

### Detailed description of the disclosure

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.
All references cited in this description are hereby deemed to be incorporated in their entirety by way of reference.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20 % or less, in particular +/-10 % or less, more in particular +/-5 % or less, even more in particular +/-1 % or less, and still more in particular +/-0.1 % or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "weight percent", "%wt" or "weight%", here and throughout the description unless otherwise defined, refers to the relative weight of the respective component based on the overall weight of the formulation.

The term "high pressure" as used herein refers to a pressure of at least 10.0 MPa or ranging from 10.0 MPa to 30.0 MPa.

In a first aspect, the present disclosure provides a method for operating a urea plant comprising a urea synthesis section, wherein the urea synthesis section comprises at least one high-pressure process line, a flushing system fluidly connected to the at least one high-pressure process line, a first high pressure flushing pump configured to direct an aqueous solution to the flushing system, at least one sensor for measuring a process variable, a second high-pressure flushing pump configured to direct an aqueous solution to the at least one sensor for measuring a process variable, characterized in that the method comprises continuously directing an aqueous solution from the second high-pressure flushing pump to the flushing system.

Urea-producing plants comprise a urea synthesis section, a recovery section, an evaporation section, and a finishing section. The urea synthesis section, recovery section, and evaporation section process aqueous solutions comprising urea, ammonium salts, such as ammonium carbamate, ammonium carbonate, and ammonium bicarbonate, and free ammonia, with different compositions.

The urea synthesis section comprises a urea synthesis reactor, and optionally a high-pressure stripper, a high-pressure carbamate condenser, a high-pressure carbamate separator, and/or a high-pressure carbamate pump.

In some embodiments, the urea synthesis section comprises one or more devices selected from the group consisting of a urea synthesis reactor, a high-pressure stripper, a high-pressure carbamate condenser, a high-pressure carbamate separator, and a high- pressure carbamate pump.

The urea synthesis reactor is configured to contain ammonia and carbon dioxide under high temperature and pressure to form a solution comprising urea, one or more ammonium salts, and free ammonia. If present, the high-pressure stripper is configured to receive the solution produced in the synthesis reactor, heat up said solution to remove some of the ammonia and ammonium salts comprised therein. The solution produced in the high-pressure stripper also comprises urea, one or more ammonium salts, and free ammonia. Simultaneously, the high-pressure stripper produces a gas stream comprising water, ammonia, and carbon dioxide. The high-pressure carbamate condenser is configured to receive the gas stream produced by the high-pressure stripper and produce an aqueous solution comprising ammonium salts and free ammonia.

The recovery section is configured to receive the solution produced by the high-pressure stripper or the synthesis device and to remove the one or more ammonium salts and ammonia comprised therein. A recovery section may contain one or more heat exchangers to remove one or more ammonium salts and ammonia from an aqueous solution. A recovery section may contain one or more carbamate condensers to condense a gas stream comprising ammonia and carbon dioxide into an aqueous solution comprising ammonia and ammonium salts. A recovery section may also contain one or more liquid/vapor separators. The solution produced by the recovery section comprises urea, in particular at least 50 weight% of urea, biuret, and optionally, one or more ammonium salts and free ammonia.

The evaporation section is configured to receive the aqueous solution produced by the recovery section and concentrate it by removing water. The evaporation section may comprise one or more evaporation units, each units being configured to produce a melt comprising urea, biuret, and optionally one or more ammonium salts and free ammonia.

The synthesis section comprises at least one high-pressure process line and a flushing system fluidly connected to the at least one high-pressure process line. The at least one high-pressure process line is a pipe configured to direct a fluid composition from one device to another under high pressure, i.e., a pressure of at least 10.0 MPa, or ranging from 10.0 MPa to 30.0 MPa. The at least one high-pressure process line is connected via a valve to a flushing system, which allows the fluid contained in the process line to be emptied to a storage or waste-disposal system when required, for example when the plant is shutdown.

The flushing system comprises one or more pipes and one or more valves.

It is important for an operator in a urea plant to obtain real-time measurements of various process variables, such as a flow, a pressure, or a temperature, at different points in the plant to ensure that the plant is running as normal. If a deviation from a predetermined value or range is detected, measures must be initiated to bring the variable back into the desired value or range. If the process deviates too much from the standard conditions, the risk for accidents, injuries to the plant staff, and damages to the plant equipment increases. So, a urea plant comprises multiple sensors, for example pressure sensors or level sensors, installed in all the sections of the plant, including the synthesis section, for measuring a process variable.

The fluid compositions in the urea synthesis section, in particular aqueous solutions comprising ammonium carbamate, can be highly corrosive. Even if the sensors are designed to operate under such high-pressure conditions and according to the design of the sensor, it is preferable to avoid the contact time between a sensor and a corrosive fluid. Therefore, the synthesis section is configured such that sensors comprised therein are continuously flushed with an aqueous solution, such as water or process condensate. A pump is required to provide the necessary solution, and this pump is conventionally called the high-pressure instrumentation flushing pump, and is referred to herein as the second high-pressure flushing pump. The second high-pressure flushing pump is fluidly connected to one or more devices and/or one or more process lines, such that it can flush sensors, in particular pressure and/or level sensors. In order to maintain, the correct process conditions, the second high-pressure flushing pump needs to pump a fluid to the working pressure of the devices and/or process lines it is connected to, for example at least 10.0 MPa, or from 15.0 MPa to 25.0 MPa.

In some embodiments, the second high-pressure flushing pump is configured to produce a flow ranging from 10 to 500 liters/hour to direct an aqueous solution to multiple sensors. The second high-pressure flushing pump is a smaller pump than the first high-pressure flushing pump and is often configured to deliver a flow ranging from 10 to 500 liters/hour. The volume of a flushing system may be ranging from a 1.0 to 10 m³. It was found that a portion of the flow delivered by the second high-pressure flushing pump can be used to continuously flow into the flushing system to keep the flushing system pressurized : if an isolation valve between the flushing system and the synthesis section is leaking, a small quantity of water (few liters) will enter into the process line, not affecting the performance of the synthesis section and keeping the flushing system free of ammonium carbamate.

Only a minor modification of the piping system is required: a pipe connecting the second high-pressure flushing pump to the flushing system was installed, the pipe comprising a valve, such as an isolation valve, also called a shut-off valve, such that the flow to the flushing system can be stopped if required. A check valve, also called non-return valve, can also be installed on the new pipe to prevent reverse flow to the second high-pressure flushing pump.

By continuously directing an aqueous solution from the second high-pressure flushing pump to the flushing system, the flushing system is kept clean, and the operating costs of the plant are only increased slightly.

The urea plant also comprises a first high-pressure flushing pump configured to direct an aqueous solution to the flushing system to drain and flush the synthesis section pipes and devices when required, for example when the plant is stopped for an extended period. Flushing and draining the synthesis section for a plant stop with only the second high-pressure flushing pump would take a long time and induce additional wear on the second pump. Instead, the urea plant may comprise a first high-pressure flushing pump configured to produce a much higher flow than the second high-pressure flushing pump, for example from 10 to 50 m³/h, from 10 to 25 m³/h, or from 10 to 15 m³/h.

In some embodiments, the at least one high-pressure process line connects the synthesis reactor to the high-pressure stripper, the high-pressure carbamate condenser to the synthesis reactor, the high-pressure carbamate condenser to the high-pressure carbamate separator, the high-pressure carbamate separator to the synthesis reactor, the high-pressure scrubber to the high-pressure carbamate condenser, the high-pressure carbamate pump to the high-pressure scrubber, or the high-pressure carbamate pump to the high-pressure carbamate condenser. Different structures of urea plants exist today depending on the technology licensor that was selected to build the plant. Stamicarbon, Saipem, Casale, and Toyo represent a large share of the existing urea plants.

In some embodiments, the synthesis section comprises more than one high-pressure process line and more than one flushing system. In some urea plants, in particular plants comprising a high-pressure stripper and a high-pressure carbamate condenser and a high pressure scrubber or a high pressure separator, more than one high-pressure process line can be found, in particular between the synthesis reactor and the high-pressure stripper, and between the high-pressure carbamate condenser and the synthesis reactor and between the synthesis reactor and the high pressure scrubber and between high pressure scrubber and high pressure carbamate condenser and between the high pressure scrubber and the high pressure separator. If a plant comprises a high-pressure carbamate pump, it also comprises a high-pressure process line between the high-pressure carbamate pump and a high-pressure device, such as the high-pressure scrubber or the high-pressure carbamate condenser.

In some embodiments, the second high-pressure flushing pump is configured to produce a flow at a pressure ranging from 10.0 to 25.0 MPa. The second high-pressure flushing pump is configured to provide a flow at a pressure equal or close to the operating pressure of the synthesis section of the plant. The operating pressure of the synthesis section may vary from one plant to another, but it is often at least 10.0 MPa, for example ranging from 15.0 to 25.0 MPa.

In some embodiments, the flushing system comprises one or more pipes.

In some embodiments, the flushing system comprises one or more flow-regulating valve.

In another aspect, the present disclosure provides an assembly comprising a flushing system, a first high-pressure flushing pump fluidly connected to the flushing system and configured to direct an aqueous solution to the flushing system, a second high-pressure flushing pump and at least one sensor for measuring a process variable in the synthesis section, wherein the flushing system comprises at least one pipe and at least one flow-regulating valve, and the second high-pressure flushing pump is fluidly connected to the flushing system and to the at least one sensor and configured to provide an aqueous solution to the flushing system and to the at least one sensor.

In another aspect the present disclosure provides a urea plant comprising a urea synthesis section as described elsewhere herein and an assembly comprising a flushing system, a first high-pressure flushing pump fluidly connected to the flushing system and configured to direct an aqueous solution to the flushing system, a second high-pressure flushing pump and at least one sensor for measuring a process variable in the synthesis section, wherein the flushing system comprises at least one pipe and at least one flow-regulating valve, and the second high-pressure flushing pump is fluidly connected to the flushing system and to the at least one sensor and configured to provide an aqueous solution to the flushing system and to the at least one sensor.

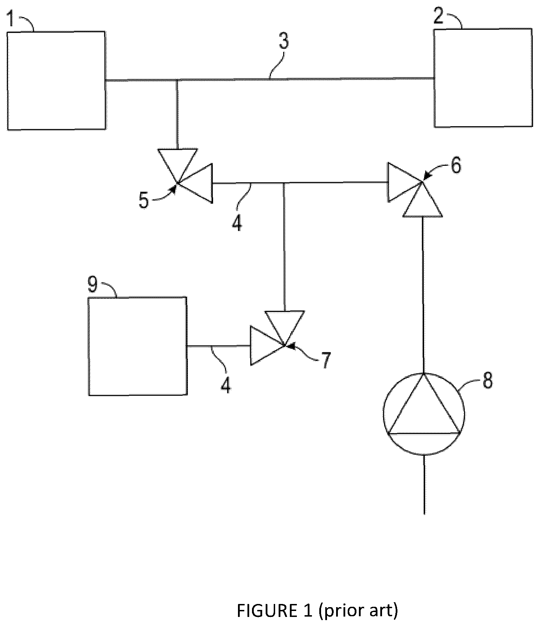

Figure 1 shows an assembly in a conventional urea plant comprising a flushing system according to the prior art. The synthesis section comprises two devices (1, 2), which may be a synthesis reactor, a high-pressure stripper, a high-pressure carbamate condenser, a high-pressure scrubber, a high-pressure separator, or a high-pressure carbamate pump. The two devices (1, 2) are fluidly connected via a high-pressure process line (3) and the high-pressure process line (3) is fluidly connected to a flushing system via an isolation valve (5). The flushing system comprises pipes (4), two valves (6, 7) and a collecting system (9). The flushing system is fluidly connected, via valve (6), to a first high-pressure pump (8) configured to deliver water or process condensate at a pressure of 200 bar and a flow of at least 10 m³/hour, when the plant needs to be stopped, and the synthesis section needs to be drained and flushed.

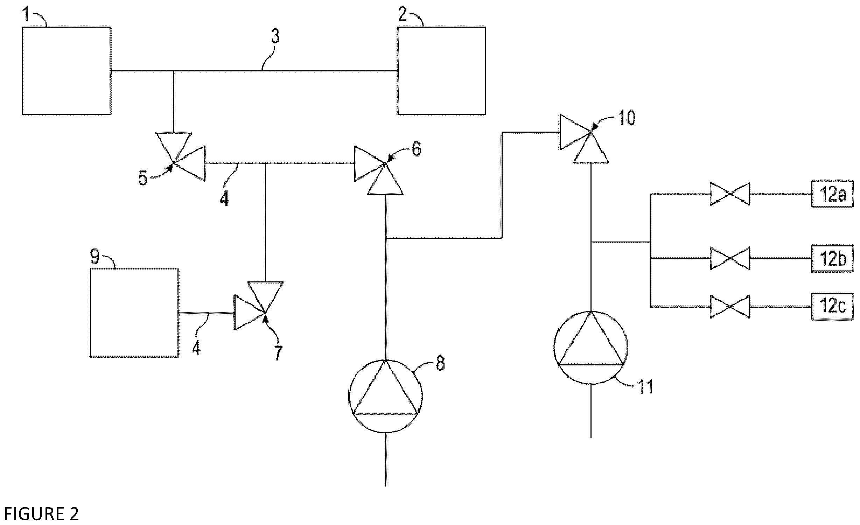

Figure 2 shows an assembly in a urea plant comprising a flushing system for performing the method according to the present disclosure. The synthesis section comprises two devices (1, 2), which may be a synthesis reactor, a high-pressure stripper, a high-pressure carbamate condenser, a high-pressure scrubber, a high-pressure separator, or a high-pressure carbamate pump. The two devices (1, 2) are fluidly connected via a high-pressure process line (3) and the high-pressure process line (3) is connected to a flushing via an isolation valve (5). The flushing system comprises pipes (4), three valves (6, 7, 10) and a collecting system (9). The flushing system is fluidly connected, via valve (6), to a first high-pressure pump (8) configured to deliver water or process condensate at a pressure of 200 bar and a flow of at least 10 m³/hour, when the plant needs to be stopped, and the synthesis section needs to be drained and flushed. The flushing system is also fluidly connected, via valve (10) to a second high-pressure flushing pump (11) configured to deliver water or process condensate at a pressure of 200 bar and a flow ranging from 10 to 100 liters/hour. The second high-pressure flushing pump (11) is also fluidly connected to several sensors (12a, 12b, and 2c) configured to measure a process variable, such as a pressure, a temperature, or a flow, in the synthesis section, and configured to direct water or process condensate to the sensors (12a, 12b, and 12c).

## Claims

1. A method for operating a urea plant comprising an urea synthesis section, wherein the urea synthesis section comprises at least one high-pressure process line, a flushing system fluidly connected to the at least one high-pressure process line, a first high-pressure flushing pump configured to direct an aqueous solution to the flushing system, at least one sensor for measuring a process variable, a second high-pressure flushing pump configured to direct an aqueous solution to the at least one sensor for measuring a process variable, **characterized in that** the method comprises continuously directing an aqueous solution from the second high-pressure flushing pump to the flushing system.

2. The method according to claim 1, wherein the urea synthesis section comprises one or more devices selected from the group consisting of a synthesis reactor, a high-pressure stripper, a high-pressure carbamate condenser, a high-pressure carbamate separator, a high-pressure scrubber and a high-pressure carbamate pump.

3. The method according to claim 2, wherein the at least one high-pressure process line connects the synthesis reactor to the high-pressure stripper, the high-pressure carbamate condenser to the synthesis reactor, the high-pressure carbamate condenser to the high-pressure carbamate separator, the high-pressure carbamate separator to the synthesis reactor, the high-pressure scrubber to the high-pressure carbamate condenser, the high-pressure carbamate pump to the high-pressure scrubber, or the high-pressure carbamate pump to the high-pressure carbamate condenser.

4. The method according to any one of claims 1 to 3, wherein the synthesis section comprises more than one high-pressure process line and more than one flushing system.

5. The method according to any one of claims 1 to 4, wherein the second high-pressure flushing pump produces a flow at a pressure ranging from 10.0 to 25.0 MPa.

6. The method according to any one of claims 1 to 5, wherein the second high-pressure flushing pump produces a flow ranging from 10 to 500 liters/hour, particularly from 10 to 100 liters/hour.

7. The method according to any one of claims 1 to 6, wherein the first high-pressure flushing pump is configured to produce a flow at a pressure ranging from 15.0 to 25.0 MPa and ranging from 10 to 50 m³/hour.

8. The method according to any one of claims 1 to 7, wherein the flushing system comprises one or more flow-regulating valves.

9. An assembly comprising a flushing system, a first high pressure flushing pump fluidly connected to the flushing system and configured to direct an aqueous solution to the flushing system, a second high-pressure flushing pump and at least one sensor for measuring a process variable in the synthesis section, wherein the flushing system comprises at least one pipe and at least one flow-regulating valve, and the second high-pressure flushing pump is fluidly connected to the flushing system and to the at least one sensor and configured to provide an aqueous solution to the flushing system and to the at least one sensor.

10. The assembly according to claim 9, wherein the second high-pressure flushing pump is configured to produce a flow at a pressure ranging from 15.0 to 25.0 MPa and/or a flow ranging from 10 to 500 liters/hour.

11. The assembly according to claim 9 or 10, wherein the first high-pressure flushing pump is configured to produce a flow at a pressure ranging from 15.0 to 25.0 MPa and/or a flow ranging from 10 to 50 m³/hour.

12. An urea plant comprising an urea synthesis section and an assembly according to any one of claims 9 to 11, wherein the synthesis section comprises at least one high-pressure process line, and wherein the flushing system is fluidly connected to the at least one high-pressure process line.

13. The urea plant according to claim 12, wherein the urea synthesis section comprises one or more devices selected from the group consisting of a urea synthesis reactor, a high-pressure stripper, a high-pressure carbamate condenser, a high-pressure carbamate separator, a high-pressure scrubber and a high- pressure carbamate pump and wherein the at least one high-pressure process line connects the urea synthesis reactor to the high-pressure stripper, the high-pressure carbamate condenser to the urea synthesis reactor, the high-pressure carbamate condenser to the high-pressure carbamate separator, the high-pressure carbamate separator to the urea synthesis reactor, the high-pressure scrubber to the high-pressure carbamate condenser, the high-pressure carbamate pump to the high-pressure scrubber, or the high-pressure carbamate pump to the high-pressure carbamate condenser.
